# EUROPÄISCHE PATENTSCHRIFT

(11) **EP 2 243 838 B1**
(45) Veröffentlichungstag und Bekanntmachung des Hinweises auf die Patenterteilung: **21.03.2012**
(21) Anmeldenummer: 10450065.7
(22) Anmeldetag: 22.04.2010
(51) Int. Cl.: C12P 5/02, C12P 3/00, C12M 1/107, C12M 1/113

(54) **Verfahren zur Herstellung von Methan durch Fermentation von Biomasse, wobei während der Versäuerung freigesetzte Wasserstoffionen an einer Kathode reduziert werden und gasförmiger Wasserstoff abgezogen wird**
Process for producing methane by fermentation of biomass, wherein hydrogen ions released during the acidification are reduced at a cathode and gasous hydrogen is removed
Procédé de production de méthane par fermentation de biomasse, dont les ions d'hydrogène libérés au cours de l'acidification sont réduits à une cathode et l'hydrogène gazeux est éliminé

(30) Priorität: 22.04.2009 AT 6132009
(43) Veröffentlichungstag der Anmeldung: 27.10.2010
(73) Patentinhaber: Joas, Elisabeth, 4840 Vöcklabruck (AT); Muhr-Kröpfel, Elisabeth, 4800 Attnang-Puchheim (AT); Pickhardt-Kröpfel, Sonja, 4800 Attnang-Puchheim (AT); Schwarzlmüller-Kröpfel, Martina, 4800 Attnang-Puchheim (AT)
(72) Erfinder: Joas, Emil, Mag., 4840 Vöcklabruck (AT)
(74) Vertreter: Hübscher, Helmut

(56) Entgegenhaltungen:
- AT-A4- 505 400
- US-A- 4 090 940
- DATABASE WPI Week 199431 Thomson Scientific, London, GB; AN 1994-251982 XP002597170 & JP 6 182397 A (TOSHIBA KK) 5. Juli 1994 (1994-07-05)

## Beschreibung

Die Erfindung bezieht sich auf ein Verfahren zur biogenen Synthese von Methan aus Biomasse, die durch eine Hydrolyse und eine Versäuerung aufgeschlossen und einer Methanogenese unterworfen wird, sowie auf eine Vorrichtung zur Durchführung des Verfahrens.

Bei der biogenen Synthese von Methan aus Biomasse wird zunächst die zerkleinerte Biomasse, die komplexe Verbindungen, wie Kohlenhydrate, Eiweiße und Fette aufweist, durch eine Hydrolyse in einfachere organische Verbindungen, z. B. Zucker, Aminosäuren und Fettsäuren, zerlegt, und zwar mit Hilfe bakterieller Enzyme, die die Biomasse auf einem biochemischen Weg abbauen. In einer anschließenden Versäuerungsphase werden diese Zwischenprodukte durch eine Acidogenese zu niederen Fettsäuren, Kohlenstoffdioxid und Wasserstoff umgesetzt, bevor in einer Acetogenese die niederen Fettsäuren zu Essigsäure, Wasserstoff und Kohlendioxid abgebaut werden. In der Methanogenese erfolgt dann die Bildung von Methan aus den während der Versäuerung erhaltenen Produkten. Diese Verfahrensschritte laufen üblicherweise in einem einzigen Reaktor oder räumlich getrennt in zwei Reaktoren ab, wobei die Hydrolyse und die Acidogenese zu einer und die Acetogenese und die Methanogenese zu einer davon getrennten Verfahrensstufe zusammengefasst werden. Unabhängig davon, ob ein ein- oder zweistufiges Verfahren eingesetzt wird, ergibt sich das Problem, dass einerseits die Versäuerung rascher als die Methanogenese abläuft und anderseits die methanogenen Bakterien empfindlich auf eine Übersäuerung reagieren, sodass die Methanogenese im Wesentlichen die zuführbare Menge an Biomasse und damit die Produktivität der Biogaserzeugung bestimmt, was insbesondere Kleinanlagen zur Biogaserzeugung unwirtschaftlich macht, wie sie im Zuge landwirtschaftlicher Betriebe erfolgreich zum Einsatz kommen könnten.

Darüber hinaus ist es bekannt (AT 505 400 B1), durch die Verknüpfung der Hydrolyse mit der Versäuerung bei gleichzeitiger Unterdrückung der anschließenden Methanogenese Wasserstoff zu gewinnen. Zu diesem Zweck werden während der Versäuerung freiwerdende Wasserstoffionen durch ein Anlegen eines elektrischen Gleichfeldes an einer Kathode gesammelt und laufend als molekularer Wasserstoff abgezogen. Die gleichzeitig freiwerdenden Sauerstoffionen sammeln sich an der Anode und werden als molekularer Sauerstoff zusammen mit dem freigesetzten Kohlendioxid ebenfalls abgezogen. Dieses bekannte Verfahren zur Gewinnung von Wasserstoff durch partiellen anaeroben Abbau biogener Stoffe kann wirtschaftlich nur für Großanlagen eingesetzt werden. Für Kleinanlagen muss daher die Biogaserzeugung im Vordergrund stehen.
Das Dokument JP6182397 offenbart ein Verfahren und eine Vorrichtung zur Herstellung von Methan durch Fermentation von Biomasse, wobei molekularer Wasserstoff in einem elektrischen Gleichfeld an einer Kathode im Methanogenese-Fermentationsgefäß generiert wird. Der molekulare Wasserstoff wird nicht gezielt gesammelt und abgezogen, sondern verbleibt als Substrat im Methanogenese-Fermentationsgefäß.
Das Dokument US4090940 offenbart ein Verfahren und eine Vorrichtung zur Herstellung von Methan durch Fermentation von Biomasse, wobei molekularer Wasserstoff in einem elektrischen Feld an einer Kathode im Methanogenese-Fermentationsgefäß generiert wird. Der molekulare Wasserstoff verbleibt als Substrat im Methanogenese-Fermentationsgefäß und/oder wird zusammen mit dem hergestellten Methan gesammelt und abgezogen, wobei er als Substrat in das Methanogenese-Fermentationsgefäß rückgeführt werden kann.

Der Erfindung liegt somit die Aufgabe zugrunde, ein Verfahren zur biogenen Synthese von Methan aus Biomasse der eingangs geschilderten Art so zu verbessern, dass die Produktionsrate insbesondere bei Kleinanlagen gesteigert werden kann.

Die Erfindung löst die gestellte Aufgabe dadurch, dass während der Versäuerung freigesetzte, überschüssige Wasserstoffionen in einem elektrischen Gleichfeld an einer Kathode gesammelt und als molekularer Wasserstoff abgezogen werden.

Da durch den laufenden Abzug von freiwerdendem Wasserstoff eine für Mikroorganismen toxische Wasserstoffkonzentration vermieden wird, kann die im Vergleich zur eigentlichen Methanogenese erheblich rascher ablaufende Acidogenese und Acetogenese für eine Produktionssteigerung genützt werden, weil durch den Abzug überschüssiger Wasserstoffionen der pH-Wert steigt und trotz der rascher ablaufenden Versäuerung vorteilhafte Bedingungen für die Methanogenese eingehalten werden können. Zusätzlich fällt molekularer Was-serstoff als Wertstoff an. Die Ableitung des Wasserstoffs aus der Versäuerungsphase kann in an sich bekannter Weise durch ein elektrisches Gleichfeld sichergestellt werden, weil sich die positiv geladenen Wasserstoffionen an der Kathode sammeln und durch Aufnahme von Elektronen in neutrale Atome übergehen, die sich zu Wasserstoffmolekülen verbinden und als Gas entweichen. In einer ähnlichen Reaktion geben die bei der Versäuerung freiwerdenden Sauerstoffionen Elektronen an eine Anode ab, sodass molekularer Sauerstoff zusammen mit dem anfallenden Kohlendioxid aus dem Substrat entweicht. Die wirksamen elektrischen Felder müssen allerdings ausreichend klein gehalten werden, um den mikrobiologischen Abbau der Spaltprodukte aus der Hydrolyse durch die Acidogenese und Acetogenese nicht zu gefährden.

Damit die Methanogenese nicht aufgrund einer zu hohen Wasserstoffkonzentration zum Erliegen kommt, wenn die Abfuhr des überschüssigen Wasserstoffs unterbrochen wird, kann in diesem Fall der einem Aufschluss durch Versäuerung unterworfenen Biomasse Kohlendioxid zugeführt werden, das aufgrund seiner Affinität zu Wasserstoff die frei werdenden überschüssigen Wasserstoffionen an sich bindet, sodass die Konzentration der Wasserstoffionen auf ein für die nachfolgende Methanogenese günstiges Maß abgesenkt werden kann. Da bei der während der Methanogenese stattfindenden Umwandlung der Essigsäure neben Methan auch Kohlendioxid anfällt, kann dieses Kohlendioxid aus dem Biogas abgetrennt und der Biomasse zugeführt werden.

Um vorteilhafte Verfahrensbedingungen sicherstellen zu können, kann von einer Vorrichtung mit einem gesonderten, zylindrischen Reaktor für die Versäuerung der durch Hydrolyse aufgeschlossenen Biomasse ausgegangen werden, wobei dieser Reaktor mit einer koaxialen, mittigen Elektrode als Anode und mit die mittige Elektrode konzentrisch umgebenden, entlang des Reaktormantels angeordneten Elektroden als Kathoden zum Aufbau eines elektrischen Gleichfeldes versehen wird, sodass die im Reaktor während der Versäuerung freiwerdenden Wasserstoffionen aufgrund des sich zwischen der mittigen Anode und den randseitigen Kathoden aufbauenden elektrischen Gleichfelds von den randseitigen Kathoden angezogen werden, während zugleich die freigesetzten Sauerstoffionen gegensinnig zur Anode wandern. Der sich im Bereich der Kathoden bildende molekulare Wasserstoff, der entlang des Reaktormantels im Substrat aufsteigt, kann in einer oberhalb der entlang des Reaktormantels angeordneten Elektroden angeordneten Ringkammer gesammelt und über eine an die Ringkammer angeschlossene Wasserstoffleitung abgezogen werden.

Die mittige Elektrode kann vorteilhaft als mit Rührflügeln versehenes Rührwerk ausgebildet werden, das nicht nur für eine gleichmäßige Verteilung des in den Reaktor geförderten Gärsubstrats sorgt, sondern auch mit entsprechenden Rührarmen Schwimmschichten zerstört, die eine laufende Entgasung des Substrats behindern.

Zur Zuführung von Kohlendioxid zum Gärsubstrat kann der Reaktor für die Versäuerung im Bodenbereich an eine Kohlendioxidleitung angeschlossen sein, sodass das Kohlendioxid den Reaktor in einem ausreichend gleichmäßigen Ausmaß von unten nach oben durchsetzt und damit vorteilhafte Voraussetzungen zur Anlagerung der freiwerdenden Wasserstoffionen schafft.

Das erfindungsgemäße Verfahren wird anhand der Zeichnung näher erklärt. Es zeigen:
- Fig. 1: eine erfindungsgemäße Vorrichtung zur biogenen Synthese von Methan aus Biomasse in einem schematischen Blockschaltbild und
- Fig. 2: einen schematischen Querschnitt durch den Reaktor für die Versäue- rung in einem größeren Maßstab.

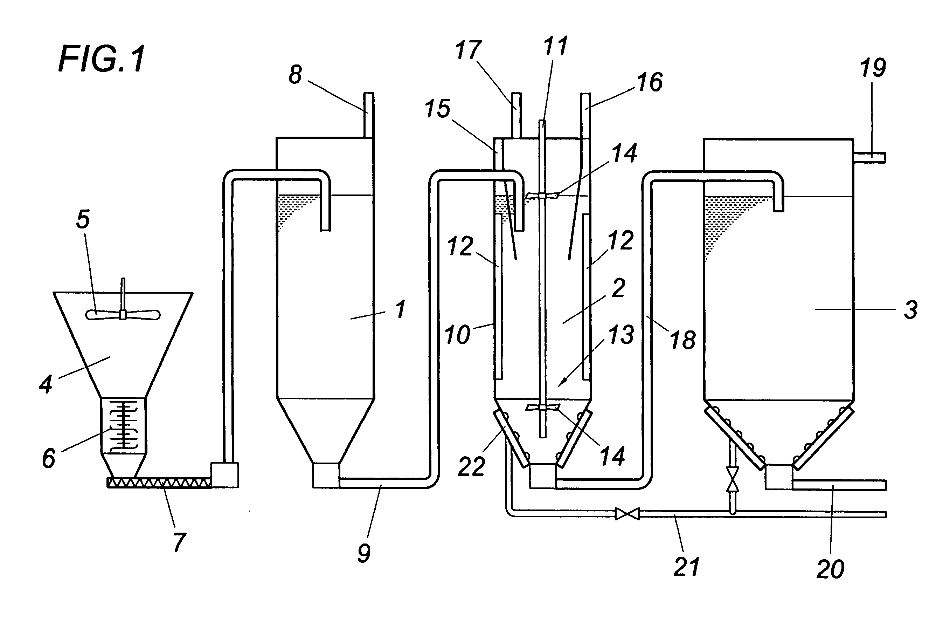

Die in der Fig. 1 dargestellte Vorrichtung zur biogenen Synthese von Methan umfasst im Wesentlichen einen Hydrolysebehälter 1, einen diesen Hydrolysebehälter 1 nachgeordneten Reaktor 2 für die Versäuerung der im Hydrolysebehälter 1 aufgeschlossenen Biomasse und einen an den Reaktor 2 für die Versäuerung angeschlossenen Reaktor 3 für die eigentliche Methanumsetzung. Die Befüllung des Hydrolysebehälters 1 erfolgt über eine Misch- und Zerkleinerungseinrichtung 4, die ein Rührwerk 5 sowie eine Schneidmühle 6 aufweist. Die der Misch- und Zerkleinerungseinrichtung 4 aufgegebenen, mit Wasser versetzten, biogenen Stoffe, die sich beispielsweise aus zwei Drittel einer landwirtschaftlichen Grünmasse und einem Drittel an Holzabfällen zusammensetzen, werden mit Hilfe einer Förderschnecke 7 in den Hydrolysebehälter 1 gefördert, wo langkettige Kohlenhydrate, Eiweiße und Fette durch Hydrolyse enzymatisch in kurzkettige wasserlösliche Bruchstücke, wie Einfachzucker, Glycerin sowie Fettsäuren und Aminosäuren, zerlegt werden. Das während der Hydrolyse freigesetzte Kohlendioxid wird aus dem Hydrolysebehälter 1 über eine Ableitung 8 abgezogen.

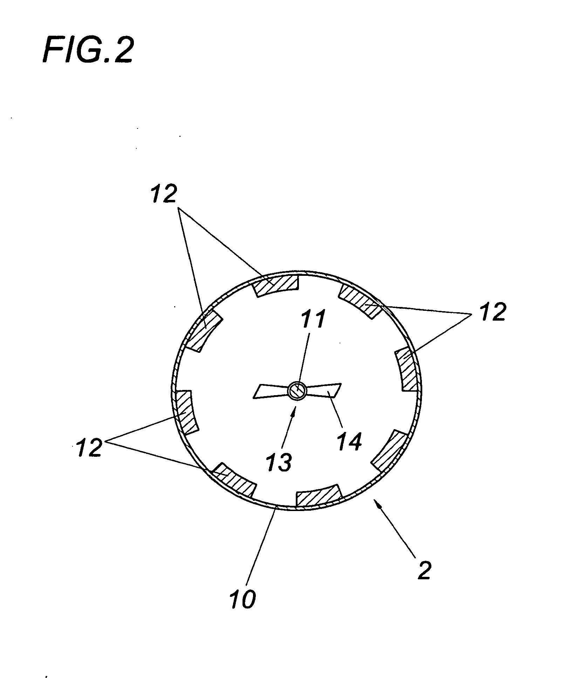

Das hydrolytisch aufgespaltene Substrat wird dann über eine Förderleitung 9 in den Reaktor 2 für die Versäuerung gefördert, um die monomeren Zwischenprodukte der Hydrolyse durch eine Acidogenese und Acetogenese in niedere Fettsäuren und dann zu Essigsäure, Wasserstoff und Kohlendioxid umzusetzen. Damit die während der Versäuerung freigesetzten Wasserstoffionen aufgrund einer höheren Konzentration nicht die nachfolgende Methanogenese behindern können, werden sie aus dem Reaktor 2 abgezogen, und zwar mit Hilfe eines elektrischen Gleichfeldes, in dem die positiven Wasserstoffionen zur Kathode wandern, während zugleich freigesetzte, negative Sauerstoffionen von der Anode angezogen werden. Zu diesem Zweck wird in den Reaktor 2, der einen zylindrischen Mantel 10 besitzt, eine mittige, zum Reaktor 2 koaxiale, als Anode wirksame Elektrode 11 eingesetzt, die konzentrisch mit als Kathode dienenden Elektroden 12 umgeben ist, wie dies insbesondere der Fig. 2 entnommen werden kann. Die Elektroden 12 sind entlang des Reaktormantels 10 angeordnet und über dessen Umfang gleichmäßig verteilt, sodass sich innerhalb des Reaktors 2 ein gleichmäßig über das Substratvolumen verteiltes, elektrisches Gleichfeld ausbilden kann. Die Elektroden 12 werden am Reaktormantel 10 ortsfest gehalten und können lediglich zu Wartungszwecken aus dem Reaktor 2 gezogen werden. Die mittige Elektrode 11 ist als Rührwerk 13 ausgebildet und weist schematisch angedeutete Rührflügel 14 auf. Oberhalb der entlang des Reaktormantels 10 vorgesehenen Elektroden 12 bildet der Reaktor 2 eine Ringkammer 15, die an eine Wasserstoffleitung 16 angeschlossen ist. Die während der Versäuerung freigesetzten Wasserstoffionen werden an den Elektroden 12 unter Aufnahme von Elektronen gesammelt und bilden in Folge molekularen Wasserstoff, der entlang der Elektroden 12 in die Ringkammer 15 aufsteigt und über die Wasserstoffleitung 16 aus dem Reaktor 2 abgezogen wird.

Die ebenfalls freigesetzten, negativen Sauerstoffionen wandern gegensinnig zu den Wasserstoffionen gegen die mittige Elektrode 11, wo sie Elektronen abgeben und molekularen Sauerstoff bilden, der mit dem freigesetzten Kohlendioxid aus dem Substrat aufsteigt und über eine Abgasleitung 17 aus dem Reaktor 2 abgezogen werden kann. Da aufgrund der Wasserstoffabfuhr die Wasserstoffionenkonzentration im Substrat auf ein für die nachfolgende Methanogenese vorteilhaftes Maß beschränkt werden kann, kann das Substrat nach der Versäuerung trotz der im Vergleich zur Methanogenese höheren Versäuerungsgeschwindigkeit dem Reaktor 3 für die Methanogenese zugefördert werden. Die hiefür vorgesehene Förderleitung ist mit 18 bezeichnet. Das Biogas, das im Wesentlichen aus Methan und Kohlendioxid zusammengesetzt ist, wird über eine Biogasleitung 19 aus dem Reaktor 3 abgezogen, während der verbleibende Faulschlamm in herkömmlicher Weise über eine Austragsleitung 20 ausgetragen wird.

Wird kein Wasserstoff benötigt, so würde die steigende Wasserstoffionenkonzentration im Reaktor 2 die anschließende Mechanisierung gefährden. Mit der Unterbrechung der Wasserstoffableitung aus dem Reaktor 2 wird daher dem Reaktor 2 Kohlendioxid über eine Kohlendioxidleitung 21 zugeführt, das über eine Verteileinrichtung 22 verteilt im Bodenbereich in den Reaktor 2 eingeleitet wird und daher das Substrat gleichmäßig durchsetzt, sodass sich die überschüssigen Wasserstoffionen an das Kohlendioxid anlagern können, wodurch die Wasserstoffionenkonzentration entsprechend gesenkt werden kann. Außerdem wird die Methanbildung aus Kohlendioxid und Wasserstoff gefördert, was zu einer Zurückdrängung der Methanbildung aus Essigsäure zugunsten der Methanbildung aus Kohlendioxid und Wasserstoff führt, die energetisch ungleich günstiger ist.

Obwohl erfindungsgemäße Vorrichtungen zur biogenen Synthese von Methan aus Biomasse erheblich kleiner als konventionelle Anlagen gebaut werden können, ermöglichen sie vergleichbare Durchsätze wegen des zulässigen hohen Versäuerungsgrads, der eine entsprechend hohe Raumbelastung ermöglicht. Der überschüssige Wasserstoff kann zusätzlich als Wertstoff abgezogen und unmittelbar, zum Beispiel für Hydrierungs- oder Reduktionsprozesse, verwendet werden.

## Patentansprüche

1. Verfahren zur biogenen Synthese von Methan aus Biomasse, die durch eine Hydrolyse und eine Versäuerung aufgeschlossen und einer Methanogenese unterworfen wird, **dadurch gekennzeichnet, dass** während der Versäuerung freigesetzte, überschüssige Wasserstoffionen in einem elektrischen Gleichfeld an einer Kathode gesammelt und als molekularer Wasserstoff abgezogen werden.

2. Verfahren nach Anspruch 1, **dadurch gekennzeichnet, dass** der einem Aufschluss durch Versäuerung unterworfenen Biomasse zum Beenden der Wasserstoffableitung Kohlendioxid zugeführt wird.

3. Vorrichtung zur biogenen Synthese von Methan aus Biomasse mit einem einem Reaktor für die Methanogenese vorgelagerten, zylindrischen Reaktor für die Versäuerung der durch Hydrolyse aufgeschlossenen Biomasse, **dadurch gekennzeichnet, dass** der Reaktor (2) für die Versäuerung eine koaxiale, mittige Elektrode (11) als Anode und die mittige Elektrode (11) konzentrisch umgebende, entlang des Reaktormantels (10) angeordnete Elektroden (12) als Kathoden zum Aufbau eines elektrischen Gleichfelds aufweist und dass oberhalb der entlang des Reaktormantels (10) angeordneten Elektroden (12) eine an eine Wasserstoffleitung (16) angeschlossene Ringkammer (15) vorgesehen ist.

4. Vorrichtung nach Anspruch 3, **dadurch gekennzeichnet, dass** die mittige Elektrode (11) als mit Rührflügeln (14) versehenes Rührwerk (13) ausgebildet ist.

5. Vorrichtung nach Anspruch 3 oder 4, **dadurch gekennzeichnet, dass** der Reaktor (2) für die Versäuerung im Bodenbereich an eine Kohlendioxidleitung (21) angeschlossen ist.

## Claims

1. Method for the biogenic synthesis of methane from biomass, which is decomposed by hydrolysis and acidification and is subjected to methanogenesis, **characterised in that** during the acidification, released excess hydrogen ions are collected in a constant electric field at a cathode and are then extracted as molecular hydrogen.

2. Method as claimed in Claim 1, **characterised in that** the biomass subjected to decomposition by acidification is supplied with carbon dioxide for terminating the derivation of hydrogen.

3. Device for the biogenic synthesis of methane from biomass, having a cylindrical reactor - mounted upstream of a reactor for methanogenesis - for the acidification of the biomass which is decomposed by hydrolysis, **characterised in that** the reactor (2) for the acidification comprises a coaxial, central electrode (11) as an anode and electrodes (12) - which concentrically surround the central electrode (11) and are disposed along the reactor jacket (10) - as cathodes for forming a constant electric field, and **in that** an annular chamber (15) connected to a hydrogen line (16) is provided above the electrodes (12) disposed along the reactor jacket (10).

4. Device as claimed in Claim 3, **characterised in that** the central electrode (11) is formed as an agitator (13) provided with agitator blades (14).

5. Device as claimed in Claim 3 or 4, **characterised in that** the reactor (2) for the acidification is connected to a carbone dioxide line (21) in the base region.

## Revendications

1. Procédé destiné à la synthèse biogène de méthane à partir de biomasse qui est désagrégée par une hydrolyse et une acidification et est soumise à une méthanogénèse, **caractérisé en ce que** des ions d'hydrogène excédentaires, libérés pendant l'acidification, sont collectés dans un champ continu électrique au niveau d'une cathode et éliminés en tant qu'hydrogène moléculaire.

2. Procédé selon la revendication 1, **caractérisé en ce que** du dioxyde de carbone est amené à la biomasse soumise à une désagrégation par acidification, pour achever l'évacuation de l'hydrogène.

3. Dispositif destiné à la synthèse biogène de méthane à partir de biomasse avec un réacteur cylindrique placé en amont d'un réacteur pour la méthanogénèse, pour l'acidification de la biomasse désagrégée par hydrolyse, **caractérisé en ce que** le réacteur (2) pour l'acidification présente une électrode centrale (11), coaxiale, en tant qu'anode, et des électrodes (12) disposées le long de l'enveloppe du réacteur (10), entourant concentriquement l'électrode centrale (11), en tant que cathodes, pour la création d'un champ continu électrique et **en ce qu'**au-dessus des électrodes (12) disposées le long de l'enveloppe du réacteur (10), il est prévu une chambre annulaire (15) raccordée à une conduite d'hydrogène (16).

4. Dispositif selon la revendication 3, **caractérisé en ce que** l'électrode centrale (11) est conçue en tant qu'agitateur (13) muni d'ailes agitatrices (14).

5. Dispositif selon la revendication 3 ou 4, **caractérisé en ce que** le réacteur (2) pour l'acidification est raccordé à une conduite de dioxyde de carbone (21) dans la zone de fond.
